# EUROPEAN PATENT SPECIFICATION

(11) **EP 2 961 463 B1**
(45) Date of publication and mention of the grant of the patent: **08.05.2019**
(21) Application number: 14705255.9
(22) Date of filing: 30.01.2014
(51) Int. Cl.: A61M 25/10, A61B 17/22

(54) **BALLOON CATHETER**
BALLONKATHETER
CATHÉTER À BALLONNET

(30) Priority: 26.02.2013 US 201361769322 P; 03.06.2013 US 201361830326 P
(43) Date of publication of application: 06.01.2016
(73) Proprietor: Cook Medical Technologies LLC, Bloomington, IN 47404 (US)
(72) Inventor: AGGERHOLM, Steen, 4660 St. Heddinge (DK); LYSGAARD, Thomas, 2680 Solroed Strand (DK)
(74) Representative: Simpson, Tobias Rutger
(86) International application number: PCT/US2014/013851
(87) International publication number: WO 2014/133708

(56) References cited:
- EP-A2- 0 200 668
- WO-A1-2010/001405
- US-A- 4 243 040
- US-A1- 2005 085 826
- US-A1- 2009 247 945

## Description

### TECHNICAL FIELD

The present invention relates generally to medical devices and more particularly to balloon catheters used to treat narrowed or obstructed portions of a body vessel. Certain aspects of the invention relate to methods of manufacturing such devices.

### BACKGROUND

Balloon catheters are widely used in the medical profession for various intraluminal procedures. One common procedure relates to the dilation of an obstructed portion of a body vessel. For example, certain body ducts that transport fluids are subject to obstruction by solid masses, or "stones", formed from crystals that separate from the transported fluid and build up within the duct. Examples of such masses include renal stones, gall stones and gastric stones. In many instances, such masses pass out of the body without the need for intervention by a physician. However, stones that cause lasting symptoms or other complications require intervention to remove the stones from the body.

Renal stones are one of the most painful of urologic disorders. Such stones form within the kidney from crystals that separate from urine. Sometimes, such stones travel down the urinary tract and are expelled from the body. In other cases, a stone may cause a blockage in the urinary tract.

Narrow tubes called ureters carry urine from the kidneys to the bladder. The bladder stores urine and eventually empties the urine into the urethra, from which it is expelled from the body. Renal stones may form in the ureters and can contain various combinations of chemicals. One type of stone contains calcium in combination with either oxalate or phosphate. Another type of stone is formed from uric acid.

Gallstones are hard masses that form in the gallbladder, a sac-like organ under the liver on the right side of the abdomen. Such masses can obstruct the bile duct, a narrow tube connecting the gallbladder to the small intestine.

Extracorporeal shock wave lithotripsy ("ESWL") is a minimally invasive treatment for the treatment of renal and gallbladder stones. In ESWL, ultrasonic sound waves that are created outside the body travel through the skin and body tissues until they hit the denser stones. The stones break down into smaller particles that can sometimes be expelled naturally from the body. Renal stones can also be removed using laser lithotripsy. This technique involves the insertion of a probe into the renal track. A cystoscope or ureteroscope is inserted into the patient's urethra, either directly or over a guide wire, and is advanced up the urinary tract to locate the target renal stone. Once the stone is located, a thin fiberoptic is introduced into a channel of the endoscope and advanced until it comes into contact with the stone. Light from a laser, for example, a holmium laser, is directed through the fiberoptic and the stone disintegrates or fragments.

In some cases, the above methods are not effective and surgery is the preferred treatment for the removal of such stones. A balloon catheter is typically used for opening the ureteral opening directly beneath the stone's location before patients undergo surgical treatment. Typically, a balloon catheter is inserted into the vessel and advanced to the occluded region. The balloon is then dilated by delivering a dilation fluid through a lumen present in the catheter shaft. Inflation of the balloon causes the exterior surface of the balloon to press against the wall of the body vessel and to expand the vessel.

The success of this procedure depends on the ability to position the inflatable balloon close to the stone blocking the vessel. However, many designs of balloon catheter include a tip portion positioned distally of the inflatable balloon. The presence of the tip limits the ability to position the balloon close to or against the stone. The use of a balloon catheter without such a tip portion offers a means of overcoming this problem. However, manufacturing balloon catheters without a tip portion presents several problems related to their complexity of manufacture.

Reference is directed to US 2005/0085826 which discloses an unfolding balloon catheter which may include an elongated shaft having a balloon that can be actuated within a body lumen to capture and retrieve an intravascular device. The balloon may be configured to radially and/or axially expand when inflated, enveloping the intravascular device. In certain embodiments, the balloon may be formed by folding the ends of a distensible member inwardly, and then bonding the ends of the distensible member to the elongated shaft to form an expandable sleeve.

Reference is further directed to US 2009/0247945 which discloses a balloon catheter system for the intraluminal advancement of conventional guidewires beyond partially or fully occluded or stenosed lesions in body passages, such as CTOs in the vasculature. Balloons are provided and reduce or minimize trauma to the vascular wall or wall of other body passages in which it is used.

Reference is further directed to US 4,243,040 which discloses an extracting device useful for removing kidney stones, gall stones and other objects from human body passages comprising a thin tubular rubber sleeve having its proximal and distal ends connected to the distal ends of outer and inner flexible tubes insertable into the body passage. The sleeve is inflatable by fluid pressure, supplied along the clearance space between the tubes to dilate the passage. The distal end of the sleeve is turned inside out and, when the sleeve is inflated, forms a soft annular flaring convex portion bulging beyond the distal end of the inner tube for sealing engagement with the stone, which is captured by suction, applied through the inner tube. Then, the inner tube is retracted, so that the suction pulls the stone within the sleeve while progressively turning the sleeve inside out around the stone to envelop the stone, for removal with the tubes. Document EP 0 200 668 A2 discloses a balloon catheter according to the preamble of claim 1.

### SUMMARY

In one aspect, the present invention provides a balloon catheter as defined by the features of claim 1.

In one embodiment, the balloon is compressed in a longitudinal direction when deflated and, upon inflation, the distal region of the balloon extends distally beyond the distal end of the catheter. In another embodiment, the catheter shaft includes an inflation lumen extending from its proximal end towards the distal end to an inflation port in fluid contact with the interior of the balloon. In yet another embodiment, the catheter shaft includes a second lumen, which is sized to accept a wire guide.

According to the invention, the distal end of the balloon has a generally flat surface perpendicular to a longitudinal axis of the inflatable balloon. The balloon can include a nylon material.

Another aspect of the present invention provides a method of manufacturing a balloon catheter as defined by the features of claim 14.

In one embodiment, the compression is such that, when the inflatable balloon is inflated, a distal region of the inflatable balloon moves distally to at least the distal end of the catheter shaft. In another embodiment, the distal region of the inflatable balloon moves distally beyond the distal end of the catheter shaft.

### BRIEF DESCRIPTION OF THE DRAWINGS

FIG. 1 is a partial side view depicting one embodiment of a balloon catheter having the balloon in a deflated condition.
FIG. 2 is a partial side view of the balloon catheter shown in FIG. 1 with the balloon in an inflated condition.
FIGS. 3 A-C illustrate one embodiment of a method of manufacturing the balloon catheter illustrated in FIGS. 1 and 2.
FIG. 4 is a flow diagram illustrating the method of manufacture shown in FIGS. 3 A-C.

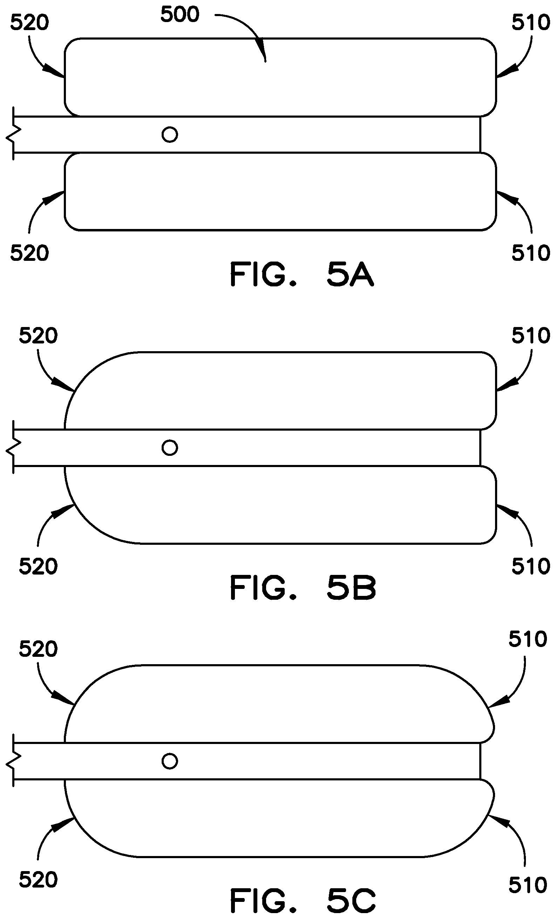

FIGS. 5 A-C illustrate a partial side view of three embodiments of a balloon catheter with the balloon in an inflated condition. FIG. 5 A illustrates an embodiment in which the proximal and distal ends of the balloon have a flattened profile. FIG. 5 B illustrates an embodiment in which the distal end of the balloon has a flattened profile while the proximal end of the balloon has a rounded or substantially conical profile. FIG. 5 C illustrates an alternative design in which the proximal and distal ends of the balloon have a rounded or substantially conical profile, hence not representing the present invention.

### DETAILED DESCRIPTION OF THE PREFERRED EMBODIMENTS DEFINITIONS

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

The uses of the terms "a" and "an" and "the" and similar references in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example") provided herein, is intended merely to better illuminate the invention and does not pose a limitation on the scope of the invention unless otherwise claimed. No language in the specification should be construed as indicating any non-claimed element as essential to the practice of the invention.

As used herein the terms "comprise(s)," "include(s)," "having," "has," "can," "contain(s)," and variants thereof, are intended to be openended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

As used herein, the term "implantable" refers to an ability of a medical device to be positioned at a location within a body, such as within a body vessel. Furthermore, the terms "implantation" and "implanted" refer to the positioning of a medical device at a location within a body, such as within a body vessel.

As used in the specification, the terms "proximal" and "distal" should be understood as being in the terms of a physician using the device. The term distal means the portion of the device which is farthest from the physician and the term proximal means the portion of the device which is nearest to the physician.

As used herein, the term "body vessel" means any body passage or lumen, including but not limited to vascular coronary or peripheral vessels, esophageal, intestinal, biliary, urethral and ureteral passages.

### TIPLESS BALLOON CATHETERS

For the purpose of promoting an understanding of the principles of the invention, reference will now be made to embodiments, some of which are illustrated in the drawings, and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

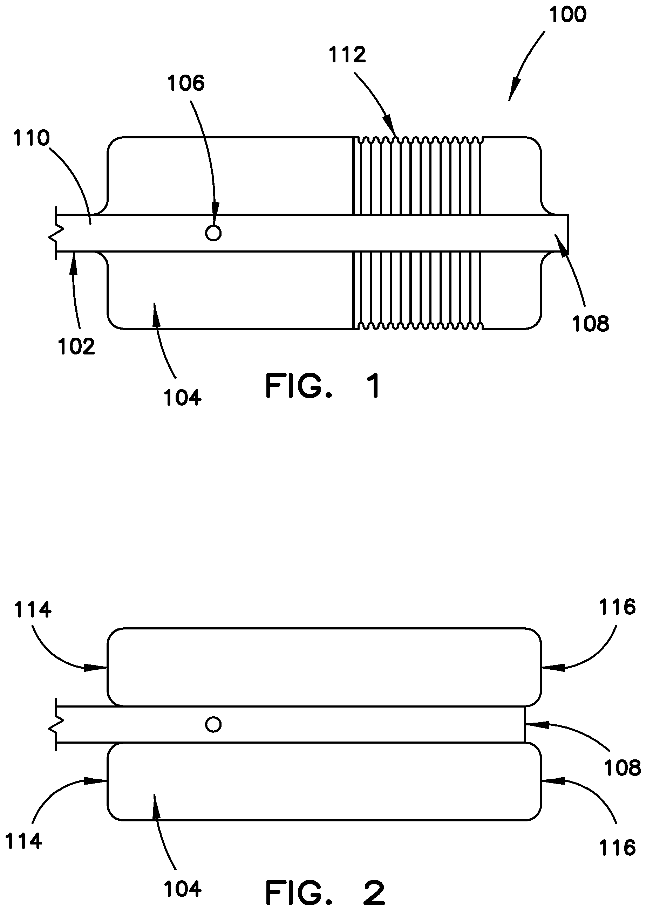

One aspect of the present invention provides balloon catheters having an inflatable balloon that extends distally to at least the distal end of the catheter shaft when the balloon is inflated (tipless balloon catheters.) Referring now to Figure 1, one embodiment of the present invention provides an implantable balloon catheter 100 including catheter shaft 102, only the distal portion of which is illustrated, and catheter balloon 104 mounted at the distal end of catheter shaft 102. Catheter shaft 102 includes an internal lumen (not shown) extending from its proximal end to inflation port 106. Inflation port 106 is in fluid contact with the interior of catheter balloon 104 and provides a means of inflating catheter balloon 104. In certain embodiments, catheter shaft may also include a wire guide lumen (not shown) for use in the delivery of the balloon catheter. Figure 1 shows catheter balloon 104 in a deflated configuration. Proximal end of catheter balloon 104 attaches to catheter shaft at position 110 and distal end of balloon 104 attaches to catheter shaft 102 at or near its distal end 108. The distance between attachment positions 108 and 110 is less than the length of catheter balloon 104. As a consequence, when deflated, catheter balloon 104 includes folded portions 112, formed as a result of the longitudinal compression of catheter balloon 102.

Figure 2 illustrates balloon catheter 100 with catheter balloon 104 in an inflated condition. As catheter balloon 104 is inflated the ends of the balloon extend outwards. In particular, distal end 116 of catheter balloon 104 extends distally beyond the distal end 108 of catheter shaft 102 while proximal end 114 of catheter balloon 104 extends proximally. As a consequence, folded portions 112 straighten until catheter balloon 104 is in a fully inflated configuration. When catheter balloon 104 is fully inflated, the distal end 116 of catheter balloon is positioned at or distally of the distal end 108 of catheter shaft 102. In this configuration, the distal end 108 of catheter shaft 102 does not obstruct the positioning of catheter balloon directly against an obstruction in a body vessel.

Such a balloon, not according to the invention, may be formed of any flexible material, such as a polyolefin, polyurethane, fluoropolymer, polyethylene, polytetrafluoroethylene (PTFE), latex, rubber, and mixtures of these materials. In one embodiment, the thickness of the wall of the balloon is approximately 0.0012 cm (0.0005 inch) thick. However, the wall of the balloon can be of any appropriate thickness provided that the thickness does not compromise properties that are important for achieving optimum performance. In various embodiments, the balloon wall thickness is within the range of approximately 0.0012 cm to 0.003 cm (0.0005 inch to 0.0012) inch thick.

According to the invention, the balloon is made from a low or non-compliant material, such as for example, nylon or polyester. A low or non-compliant balloon will increase in diameter by up to a maximum of about 5% of its normal diameter in response to increasing the pressure for inflating the balloon to between about 5 to 50 atmospheres. Alternatively however not according to the invention, the balloon may be made from a hybrid or highly compliant material where the diameter of the balloon may increase as much as about 40% during inflation. The hybrid or highly compliant balloon may proportionally increase in diameter in response to increases in inflation pressure which may allow for fewer balloon sizes to be used to treat a wider range of vessel diameters.

In various embodiments, the rated burst pressure (RBP) of the balloon is equal to or greater than about 1013, 1520, 2026, 2533, 3040, 3546, 4053, 4560 or 5066 kPa (10, 15, 20, 25, 30, 35, 40, 45 or 50 atmospheres). RBP is the statistically-determined maximum pressure to which a balloon may be inflated without rupturing. There is a 95% confidence that 99.9% of the balloons will not burst at or below the RBP upon single inflation.

The balloon can be a reinforced balloon. For example, the balloon can be reinforced by incorporating a sleeve or other reinforcing member into or onto the balloon. Examples of such reinforced balloons and their methods of manufacture are disclosed in co-pending U.S. patent application serial number 13/784,028 to Aggerholm and Lysgaard.

In one embodiment, a reinforcing member is wound around the balloon. At least one other reinforcing member may also be wound around the balloon at an angle that crosses the first reinforcing member. In other embodiments, the reinforcing member is at least partially embedded within a layer of the balloon wall. The reinforcing member may be in the form of a woven or knitted mesh of threads, in which the warp and weft fibers may extend along the longitudinal and transverse axes of the balloon. The reinforcing member can have other structures, including a coil extending helically along the balloon or a punctured or apertured sleeve of strengthening material, for instance. In one embodiment, the reinforcing member extends along the entire length of the balloon, including a generally cylindrical body portion of the balloon and the end portions either side of the body portion, preferably up to where the balloon is attached to the catheter.

The reinforcing member can be formed from a mesh of fibrous material and can be made of any suitable material including polymer, metal or metal alloy, natural fiber; one example being a polyamide such as nylon, ultra-high molecular weight polyethylene fiber such as DYNEEMA™, graft materials, suture materials and mixtures of at least two of these materials.

For example, the reinforcing member can be attached to the balloon with an adhesive or by heat fusing the reinforcing member to the balloon wall. In other embodiments, the reinforcing member is incorporated at least partially into a layer of the balloon wall. In one embodiment, the reinforced balloon is formed by a reflow process as described in co-pending U.S. patent application serial number 13/784,028 to Aggerholm and Lysgaard. For example, the balloon may be formed from raw tubing, which in one embodiment is a co-extrusion of at least a first and a second layer. The first, outer, layer is a layer of polymer material which eventually forms outer layer of the balloon. In one embodiment, the second layer forms an inner layer of the balloon. However, in other embodiments, one or more additional layers are present in the co-extrusion. In other embodiments, two or more separate layers of raw tubing, placed one inside the other, are used to form the balloon.

For example, the balloon may be manufactured by placing the raw tubing in a mold as described in co-pending U.S. patent application serial number 13/784028 to Aggerholm and Lysgaard. Here, the reinforcing member, if present, is positioned within the mold chamber prior to the insertion of the raw tubing. The raw tubing is then placed in the mold and is held tightly at the ends of the mold chamber as the mold is heated and inflation pressure fed into the lumen of the innermost raw tubing, causing the raw tubing to expand radially outwardly. The raw tubing is heated to a temperature sufficient to cause the outer layer to soften or melt. Thus, as the raw tubing expands under the inflation pressure towards the walls of the mold chamber, the reinforcing member becomes embedded, at least partially, within the material of the outermost raw tubing and thereby eventually within the outer layer of the subsequently formed balloon. Of course, when the balloon is formed from two or more separate layers of raw tubing, the reinforcing member may be placed between two of these layers so that the reinforcing member is incorporated between these layers, or at least partially within a least one of these layers.

During the molding process, the inner layer(s) of the raw tubing will likewise expand and act to press the outer layer radially outwardly as the result of inflation pressure within the lumen of the innermost layer of raw tubing. The mold is heated to a temperature sufficient to cause the inner layer(s) to soften or melt, and thereby bond together and with the outermost layer. The balloon can hence be formed within the mold by a single step manufacturing process, in contrast to methods which require subsequent manufacture and assembly steps to form multiple layers and specifically also to attach further components to the formed balloon.

In one embodiment, the temperature of the mold is subsequently raised to the heat set temperature of at least one of the layers of the balloon wall. When the material forming this layer is heated to its heat set temperature whilst it is stretched, for example by inflation within the mold, the material becomes fixed such that when inflation pressure is removed, the material maintains its size and form rather than returning to its pre-inflated size and form. In certain embodiments, the catheter shaft of the balloon catheters disclosed herein includes one or more radiopaque and/or echogenic markers to assist in the positioning of the balloon within the body vessel of a patient. In other embodiments, the balloon is inflated with an inflation fluid including a contrast medium. In yet other embodiments, a radiopaque and/or an echogenic material is incorporated into the structure of the balloon. For example, balloons containing such materials and methods of their manufacture are described in U.S. Patent Publication Number US1013/0053770, published February 28, 2013, to Aggerholm, and in co-pending U.S. patent application serial number 13/784028 to Aggerholm and Lysgaard.

The balloon wall may contain the radiopaque and/or echogenic material. In certain embodiments, the balloon includes a layer containing the radiopaque and/or echogenic material(s) and a layer containing a sleeve/ reinforcing member. In other embodiments, the sleeve/reinforcing member itself is echogenic. For example, the balloon wall may include at least two layers, one of which is formed from a polymer incorporating the radiopaque and/or echogenic material, while the other layer is formed from a polymer which may incorporate a sleeve/ reinforcing member. In such embodiments, the layer containing the radiopaque and/or echogenic material(s) may be positioned within or outside the layer including the sleeve/reinforcing member. In other embodiments, the balloon does not include a sleeve/reinforcing member. In certain embodiments, the radiopaque and/or echogenic material is one or more of: tungsten, gold, silver, carbon, platinum, palladium, barium or bismuth. Barium and bismuth are radiopaque; whereas tungsten, gold, platinum and palladium are both radiopaque and echogenic. Other echogenic materials include PVC and fluorpolymers. These materials can provide good radiopacity, and/or echogenicity, and are biocompatible. The radiopaque and/or echogenic material(s) may be in the form of powder, granules, pellets or fragments dispersed throughout the layer. Tungsten is the most preferred material as this material has very good performance even when used in small amounts. Materials which are solely echogenic can be seen by fluoroscopy techniques. In certain embodiments, the balloon layer containing the radiopaque/echogenic material(s) can include between 50 and 90% or between 60 and 80% or between 65 and 85% or between 75 and 85% by weight of radiopaque/echogenic material(s). In other embodiments, the layer incorporating the radiopaque/echogenic material(s) is unable to withstand the levels of inflation pressure to which the balloon is subjected to during its medical use. In such embodiments, a second layer, without the radiopaque/echogenic material(s) is included to provide support during inflation and use of the balloon.

One embodiment provides a balloon having two layers. The outer layer is formed from a polymer material including a reinforcing member at least partially incorporated within while the inner layer is formed from a polymer material containing tungsten. The balloon may be formed using the molding process described above. The raw tubing may be a coextruded tubing including a layer incorporating tungsten and a layer without tungsten. In another embodiment, two separate layers of raw tubing, one positioned within the other are used in the molding process. One of these raw tubing layers incorporates tungsten while the other does not.

Figures 5 A-C illustrate three alternative embodiments of the distal portion of a tipless balloon catheter. These embodiments differ in the shape of the distal and proximal end portions of the catheter balloon. In the embodiment, according to the present invention, illustrated in Figure 5 A, both the distal end 510 and the proximal end 520 of the balloon 500 have a flattened profile. In this embodiment, the ends of the inflatable balloon have a generally flat surface perpendicular to a longitudinal (proximal-distal) axis of the inflatable balloon. Figure 5 B illustrates an embodiment, also according to the present invention, in which the distal end 510 of the catheter balloon has a flattened profile while the proximal end 520 of the balloon has a rounded or substantially conical profile. Figure 5 C illustrates an embodiment, not according to the present invention, in which the proximal end 520 and distal end 510 of the balloon have a rounded or substantially conical profile.

In certain embodiments, the distal end of the catheter balloon is shaped such that, when fully inflated, at least 70, 75, 80, 85, 90, 97, 98 or 99 percentage of the distal surface of the catheter balloon is positioned perpendicular to the longitudinal axis of the catheter balloon such that, when the inflated balloon is positioned against a flat surface perpendicular to the longitudinal axis of the balloon catheter, the percentages of the distal surface of the catheter balloon listed above are in contact with the flat surface.

While the embodiment illustrated in FIGS. 1 and 5may include a wire guide lumen that extends along the entire length of the catheter from the proximal end to the distal end, it is understood that the present embodiments may also include alternative lumen configurations. For example, the wire guide lumen may extend along only a portion of the length of the catheter. Indeed, over-the-wire, rapid exchange, short wire and intraductal exchange type delivery systems are contemplated and considered to be within the scope of the invention. A rapid exchange balloon catheter includes a proximal port for the wire guide lumen that is not located at or near the proximal end of the catheter shaft. It is instead located at an intermediate location along the catheter shaft. Examples of such alternative lumen configurations are illustrated in U.S. Publication Numbers 2011/0160834 (to Aggerholm) and 2009/0149808 (to Hansen and Aggerholm).

### METHODS OF MANUFACTURIING A BALLOON CATHETER

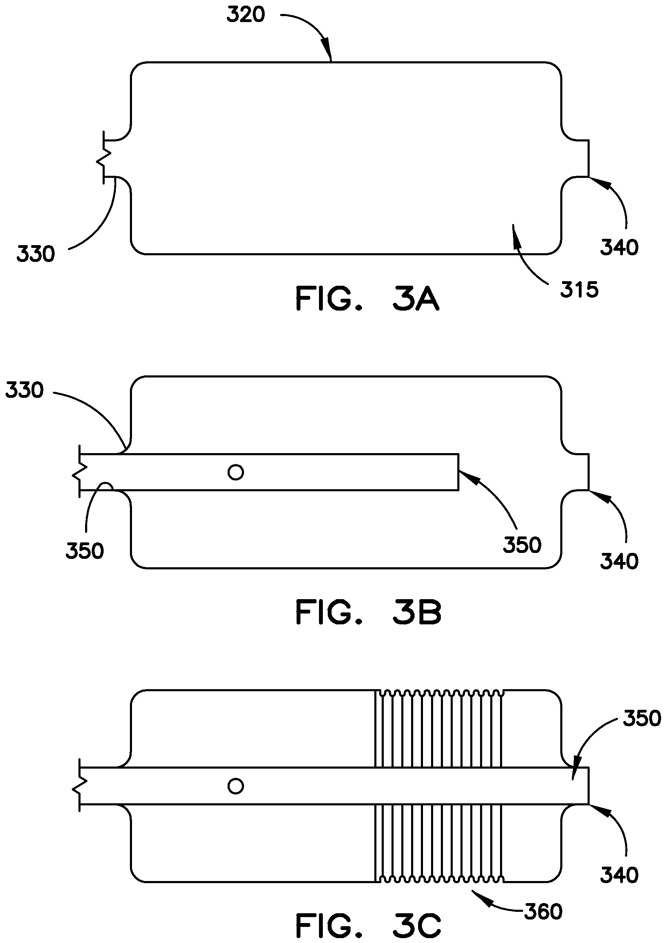

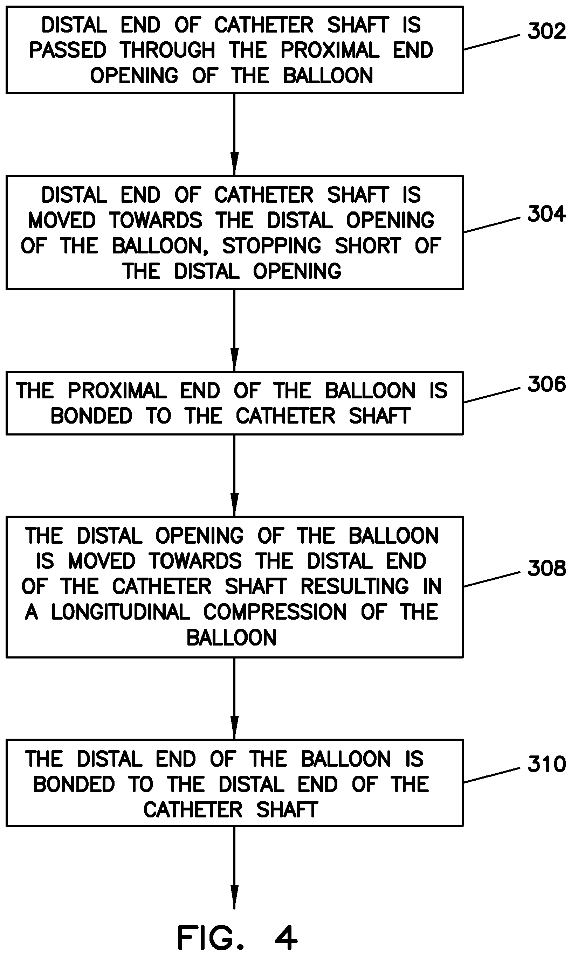

Another aspect of the present invention provides methods of manufacturing a tipless balloon catheter. Figures 3 A-C and the flow diagram shown in Figure 4 illustrate one such method of manufacturing such a balloon catheter. Figure 3 A illustrates an inflatable balloon 315 having a proximal end opening 330, a distal end opening 340 and a center portion 320. In one embodiment, the balloon is manufactured using a blow molding process including heating a material, such as a resin, within a mold to produce the balloon. The material may be heated via heaters or viscous dissipation, e.g., working of the material through the molding process, or any other suitable means for heating the material. Any polymer suitable for blow molding may also be used.

Turning now to Figure 3 B, balloon catheter shaft 350 is inserted through proximal opening 350 and advanced so that its distal end 350 is positioned proximally of distal opening 340. The proximal opening 330 of balloon 315 is then sealed to catheter shaft 350. The balloon may be attached by being hot melted, adhesively bonded or solvent fused to the catheter shaft 350. Any other suitable means known to those skilled in the art may also be used to attach the balloon to the shaft.

After attaching the proximal end of balloon 315 to the catheter shaft, the distal end 350 of balloon 315 is moved proximally to engage the distal end 340 of catheter shaft 350 and then sealed to the catheter shaft using one of the sealing methods described above. Figure 3 C shows the balloon catheter after this step is accomplished. The balloon is shown to include folded region 360, which accommodate the excess longitudinal dimension of the balloon.

### METHODS OF TREATING AN OCCLUDED BODY VESSEL

Another aspect, not according to the invention, provides a method for treating an occluded vessel using one of the embodiments of the balloon catheter disclosed herein. In one embodiment, the method includes the steps of positioning a distal end of the catheter shaft of a balloon catheter at an occluded or narrowed portion of the vessel and inflating the inflatable balloon by pumping saline or another inflation medium into the interior of the balloon. Upon inflation, the distal region of the inflatable balloon moves distally against the occluded region of the vessel and pushes against the occlusion and also against the vessel wall immediately adjacent to the obstruction.

Although the invention has been described and illustrated with reference to specific illustrative embodiments thereof, it is not intended that the invention be limited to those illustrative embodiments. Those skilled in the art will recognize that variations and modifications can be made without departing from the true scope of the invention as defined by the claims that follow. It is therefore intended to include within the invention all such variations and modifications as fall within the scope of the appended claims and equivalents thereof.

## Claims

1. A balloon catheter comprising:
an inflatable balloon (104) having an unfolded longitudinal dimension and comprising a proximal end, a middle region, a distal end and a longitudinal axis extending from the proximal end to the distal end, wherein the inflatable balloon is a low compliant or non-compliant balloon, and
a catheter shaft (102) having a distal end,
wherein the distal end of the inflatable balloon attaches to the distal end of the catheter and wherein the proximal end of the inflatable balloon attaches to the catheter shaft at a distance from the distal end of the inflatable balloon that is less than the unfolded longitudinal dimension of the inflatable balloon;
**characterised in that** the inflatable balloon is shaped such that, when fully inflated, at least 80 percentage of a surface of the distal end is positioned perpendicular to the longitudinal axis.

2. The balloon catheter of claim 1, wherein the balloon is compressed in a longitudinal direction when deflated.

3. The balloon catheter of claim 1, wherein a distal region of the balloon extends distally beyond the distal end of the catheter upon inflation.

4. The balloon catheter of claim 1, wherein the catheter shaft comprises an inflation lumen extending from its proximal end towards the distal end and in fluid contact with an interior of the inflatable balloon.

5. The balloon catheter of claim 4, wherein the catheter shaft further comprises a second lumen, wherein the second lumen is sized to accept a wire guide.

6. The balloon catheter of claim 1, wherein the distal end of the inflatable balloon comprises a generally flat surface perpendicular to a longitudinal axis of the inflatable balloon.

7. The balloon catheter of claim 1, wherein the catheter shaft comprises a radiopaque marker.

8. The balloon catheter of claim 1, wherein the inflatable balloon comprises a nylon.

9. The balloon catheter of claim 1, wherein the inflatable balloon is a non-compliant balloon.

10. The balloon catheter of claim 1, wherein the balloon comprises a material selected from the group consisting of a radiopaque material, an echogenic material and a combination thereof.

11. The balloon catheter of claim 10, wherein the material is contained within a wall of the balloon.

12. The balloon catheter of claim 1, wherein the balloon is a reinforced balloon.

13. The balloon catheter of claim 1, wherein a wall of the balloon comprises a layer comprising tungsten and a layer comprising a reinforcing member

14. A method of manufacturing a balloon catheter comprising:
inserting a distal end of a catheter shaft into a proximal end of an inflatable balloon, wherein the inflatable balloon comprises the proximal end, a middle region, a distal end and a longitudinal axis extending from the proximal end to the distal end, wherein the inflatable balloon is a low compliant or non-compliant balloon and wherein the inflatable balloon is shaped such that, when fully inflated, at least 80 percentage of a surface of the distal end is positioned perpendicular to the longitudinal axis,
advancing the distal end of the catheter shaft towards the distal end of the inflatable balloon to a position proximal of the distal end to the inflatable balloon,
bonding the proximal end of the inflatable balloon to the catheter shaft while maintaining the distal end of the catheter shaft at the position proximal of the distal end to the inflatable balloon, and
bonding the distal end of the inflatable balloon to the distal end of the catheter shaft, whereby the middle region of the inflatable balloon is compressed along a distal-proximal axis.

15. The method of claim 14, wherein the compression is such that, when the inflatable balloon is inflated, a distal region of the inflatable balloon moves distally to at least the distal end of the catheter shaft or beyond the distal end of the catheter shaft.

## Patentansprüche

1. Ballonkatheter, umfassend:
einen aufblähbaren Ballon (104) mit einer entfalteten Längsabmessung und umfassend ein proximales Ende, einen mittleren Bereich, ein distales Ende und eine Längsachse, die von dem proximalen Ende zu dem distalen Ende verläuft, wobei der aufblähbare Ballon ein wenig nachgiebiger oder nicht nachgiebiger Ballon ist, und
einen Katheterschaft (102) mit einem distalen Ende, wobei das distale Ende des aufblähbaren Ballons an dem distalen Ende des Katheters befestigt ist und wobei das proximale Ende des aufblähbaren Ballons in einem Abstand von dem distalen Ende des aufblähbaren Ballons, der kleiner als die entfaltete Längsabmessung des aufblähbaren Ballons ist, an dem Katheterschaft befestigt ist;
**dadurch gekennzeichnet, dass** der aufblähbare Ballon so geformt ist, dass, wenn vollständig aufgebläht, wenigstens 80 Prozent der Oberfläche des distalen Endes senkrecht zu der Längsachse angeordnet sind.

2. Ballonkatheter gemäß Anspruch 1, wobei der Ballon, wenn entleert, in Längsrichtung komprimiert ist.

3. Ballonkatheter gemäß Anspruch 1, wobei der distale Bereich des Ballons bei Aufblähen distal über das distale Ende des Katheters hinausragt.

4. Ballonkatheter gemäß Anspruch 1, wobei der Katheterschaft ein Aufblählumen umfasst, das von seinem proximalen Ende zu dem distalen Ende verläuft und in Fluidkontakt mit einem Innenraum des aufblähbaren Ballons steht.

5. Ballonkatheter gemäß Anspruch 4, wobei der Katheterschaft ferner ein zweites Lumen umfasst, wobei das zweite Lumen dafür bemessen ist, einen Führungsdraht aufzunehmen.

6. Ballonkatheter gemäß Anspruch 1, wobei das distale Ende des aufblähbaren Ballons eine allgemein flache Oberfläche senkrecht zu der Längsachse des aufblähbaren Ballons umfasst.

7. Ballonkatheter gemäß Anspruch 1, wobei der Katheterschaft einen strahlenundurchlässigen Marker umfasst.

8. Ballonkatheter gemäß Anspruch 1, wobei der aufblähbare Ballon ein Nylon umfasst.

9. Ballonkatheter gemäß Anspruch 1, wobei der aufblähbare Ballon ein nicht nachgiebiger Ballon ist.

10. Ballonkatheter gemäß Anspruch 1, wobei der Ballon ein Material ausgewählt aus der Gruppe bestehend aus einem strahlenundurchlässigen Material, einem echogenen Material und einer Kombination davon umfasst.

11. Ballonkatheter gemäß Anspruch 10, wobei das Material in einer Wand des Ballons enthalten ist.

12. Ballonkatheter gemäß Anspruch 1, wobei der Ballon ein verstärkter Ballon ist.

13. Ballonkatheter gemäß Anspruch 1, wobei die Wand des Ballons eine Schicht umfasst, die Wolfram umfasst, und eine Schicht, die ein Verstärkungsmaterial umfasst.

14. Verfahren zur Herstellung eines Ballonkatheters, umfassend:
Einführen eines distalen Endes eines Katheterschafts in ein proximales Ende eines aufblähbaren Ballons, wobei der aufblähbare Ballon das proximale Ende, einen mittleren Bereich, ein distales Ende und eine Längsachse, die von dem proximalen Ende zu dem distalen Ende verläuft, umfasst, wobei der aufblähbare Ballon ein wenig nachgiebiger oder nicht nachgiebiger Ballon ist und wobei der aufblähbare Ballon so geformt ist, dass, wenn vollständig aufgebläht, wenigstens 80 Prozent der Oberfläche des distalen Endes senkrecht zu der Längsachse angeordnet sind,
Vorschieben des distalen Endes des Katheterschafts in Richtung auf das distale Ende des aufblähbaren Ballons bis zu einer Position proximal bezogen auf das distale Ende des aufblähbaren Ballons,
Befestigen des proximalen Endes des aufblähbaren Ballons an dem Katheterschaft, während das distale Ende des Katheterschafts an der Position proximal bezogen auf das distale Ende des aufblähbaren Ballons gehalten wird, und Befestigen des distalen Endes des aufblähbaren Ballons an dem distalen Ende des Katheterschafts, wodurch der mittlere Bereich des aufblähbaren Ballons entlang einer distal-proximal-Achse komprimiert wird.

15. Verfahren gemäß Anspruch 14, wobei die Kompression so ist, dass sich, wenn der aufblähbare Ballon aufgebläht wird, ein distaler Bereich des aufblähbaren Ballons distal bis zu wenigstens dem distalen Ende des Katheterschafts oder über das distale Ende des Katheterschafts hinaus bewegt.

## Revendications

1. Cathéter à ballonnet comprenant :
un ballonnet gonflable (104) possédant une dimension longitudinale dépliée et comprenant une extrémité proximale, une région médiane, une extrémité distale et un axe longitudinal s'étendant depuis l'extrémité proximale vers l'extrémité distale, le ballonnet gonflable étant un ballonnet faiblement adaptatif ou non adaptatif, et
une tige (102) de cathéter ayant une extrémité distale, dans lequel l'extrémité distale du ballonnet gonflable se fixe à l'extrémité distale du cathéter et dans lequel l'extrémité proximale du ballonnet gonflable se fixe à la tige de cathéter à une distance donnée de l'extrémité distale du ballonnet gonflable qui est inférieure à la dimension longitudinale dépliée du ballonnet gonflable ;
**caractérisé en ce que** le ballonnet gonflable est formé de sorte que, lorsqu'il est entièrement gonflé, au moins 80 pour cent d'une surface de l'extrémité distale soient positionnés perpendiculairement à l'axe longitudinal.

2. Cathéter à ballonnet selon la revendication 1, dans lequel le ballonnet est comprimé dans une direction longitudinale lorsqu'il est dégonflé.

3. Cathéter à ballonnet selon la revendication 1, dans lequel une région distale du ballonnet s'étend distalement au-delà de l'extrémité distale du cathéter lors du gonflage.

4. Cathéter à ballonnet selon la revendication 1, dans lequel la tige de cathéter comprend une lumière de gonflage s'étendant depuis son extrémité proximale vers l'extrémité distale et en contact fluidique avec un intérieur du ballonnet gonflable.

5. Cathéter à ballonnet selon la revendication 4, dans lequel la tige de cathéter comprend en outre une seconde lumière, la seconde lumière étant dimensionnée pour accepter un guide-fil.

6. Cathéter à ballonnet selon la revendication 1, dans lequel l'extrémité distale du ballonnet gonflable comprend une surface généralement plate perpendiculaire à un axe longitudinal du ballonnet gonflable.

7. Cathéter à ballonnet selon la revendication 1, dans lequel la tige de cathéter comprend un marqueur radio-opaque.

8. Cathéter à ballonnet selon la revendication 1, dans lequel le ballonnet gonflable comprend du nylon.

9. Cathéter à ballonnet selon la revendication 1, dans lequel le ballonnet gonflable est un ballonnet non adaptatif.

10. Cathéter à ballonnet selon la revendication 1, dans lequel le ballonnet comprend un matériau sélectionné dans le groupe consistant en un matériau radio-opaque, un matériau échogène et une combinaison de ceux-ci.

11. Cathéter à ballonnet selon la revendication 10, dans lequel le matériau est contenu dans une paroi du ballonnet.

12. Cathéter à ballonnet selon la revendication 1, dans lequel le ballonnet est un ballonnet renforcé.

13. Cathéter à ballonnet selon la revendication 1, dans lequel une paroi du ballonnet comprend une couche comprenant du tungstène et une couche comprenant un élément de renfort.

14. Procédé de fabrication d'un cathéter à ballonnet consistant à :
introduire une extrémité distale d'une tige de cathéter dans une extrémité proximale d'un ballonnet gonflable, le ballonnet gonflable comprenant l'extrémité proximale, une région médiane, une extrémité distale et un axe longitudinal s'étendant depuis l'extrémité proximale vers l'extrémité distale, le ballonnet gonflable étant un ballonnet faiblement adaptatif ou non adaptatif et le ballonnet gonflable étant formé de sorte que, lorsqu'il est entièrement gonflé, au moins 80 pour cent d'une surface de l'extrémité distale soient positionnés perpendiculairement à l'axe longitudinal,
faire avancer l'extrémité distale de la tige de cathéter vers l'extrémité distale du ballonnet gonflable vers une position proximale de l'extrémité distale vers le ballonnet gonflable,
lier l'extrémité proximale du ballonnet gonflable vers la tige de cathéter tout en maintenant l'extrémité distale de la tige de cathéter au niveau de la position proximale de l'extrémité distale vers le ballonnet gonflable, et
lier l'extrémité distale du ballonnet gonflable à l'extrémité distale de la tige de cathéter, moyennant quoi la région médiane du ballonnet gonflable est comprimée le long d'un axe distal-proximal.

15. Procédé selon la revendication 14, dans lequel la compression est telle que, lorsque le ballonnet gonflable est gonflé, une région distale du ballonnet gonflable se déplace de façon distale vers au moins l'extrémité distale de la tige de cathéter ou au-delà de l'extrémité distale de la tige de cathéter.
